# EUROPÄISCHE PATENTSCHRIFT

(11) **EP 0 888 280 B1**
(45) Veröffentlichungstag und Bekanntmachung des Hinweises auf die Patenterteilung: **29.08.2001**
(21) Anmeldenummer: 97903297.6
(22) Anmeldetag: 17.02.1997
(51) Int. Cl.: C07C 67/343, C07C 69/734

(54) **VERFAHREN ZUR HERSTELLUNG VON 2-(2-METHYLPHENYL)-3-METHOXYACRYLSÄURE-METHYLESTER**
PROCESS FOR PREPARING 2-(2-METHYLPHENYL)-3-METHOXYACRYLIC ACID METHYLESTER
PROCEDE DE PREPARATION DE METHYLESTER D'ACIDE 2-(2-METHYLPHENYLE)-3-METHOXYACRYLIQUE

(30) Priorität: 17.02.1996 DE 19605901
(43) Veröffentlichungstag der Anmeldung: 07.01.1999
(73) Patentinhaber: BASF AKTIENGESELLSCHAFT, 67056 Ludwigshafen (DE)
(72) Erfinder: KEIL, Michael, D-67251 Freinsheim (DE); WAHL, Josef, D-67105 Schifferstadt (DE)
(86) Internationale Anmeldenummer: EP9700728
(87) Internationale Veröffentlichungsnummer: WO9730020

(56) Entgegenhaltungen:
- WO-A-94/05622

## Beschreibung

Die vorliegende Erfindung betrifft ein Verfahren zur Herstellung von 2-(2-Methylphenyl)-3-methoxyacrylsäure-methylester (I) 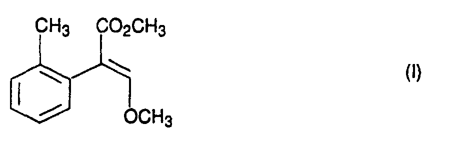 durch Formylierung von 2-Methylphenylessigsäure-methylester (II) 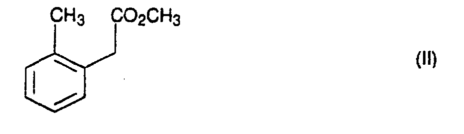 in einem inerten Lösungsmittel in Gegenwart einer Base, und anschließende Methylierung des gebildeten Enolats III 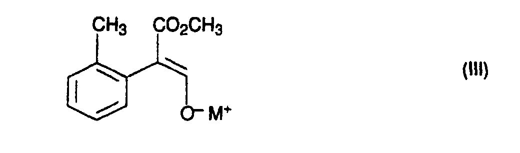 in dem M⁺ für ein Alkalimetall-kation steht.

Die Herstellung von 2-(2-Methylphenyl)-3-methoxyacrylsäure-methylester ist vielfach in der Literatur beschrieben (vgl. EP-A 178 826, EP-A 203 606, EP-A 226 917, EP-A 473 980 und WO-A 94/05,622).

EP-A 178 826 beschreibt für die Formylierung die Verwendung von Dimethylformamid als inertes Lösungsmittel und Natrium-hydrid als Base. Die Formylierung erfolgt bei Temperaturen von 0-5°C. Die anschließende Methylierung wird in Dimethylformamid mit Dimethylsulfat in Gegenwart von Kalium-carbonat durchgeführt. Ähnliche Umsetzungen sind aus EP-A 243 012, EP-A 273 572, EP-A 329 011 und EP-A 480 795 bekannt.

Gemäß EP-A 203 606 und EP-A 226 917 erfolgt die Formylierung in Ether in Gegenwart von Natrium-hydrid bei der Siedetemperatur des Gemisches. Die anschließende Methylierung wird in Aceton mit Dimethylsulfat in Gegenwart von Kalium-carbonat durchgeführt.

Nach einer weiteren Variante erhält man 3-Alkoxy-2-heteroazolylamino-acrylsäureester durch Formylierung in Dimethylformamid in Gegenwart von Alkalimetall-alkoholaten bei 0-5°C und anschließende Methylierung mit Dimethylsulfat (EP-A 473 980).

Außerdem wird in der WO-A 94/05, 622 die Herstellung von 2-(2-Methylphenyl)-3-methoxyacrylsäure-methylester (I) durch Formylierung in Kohlenwasserstoffen in Gegenwart eines Phasentransfer-Katalysators beschrieben.

Diese bekannten Verfahren haben den Nachteil, daß
- Durchführung und Aufarbeitung zum Teil sehr umständlich sind, so daß eine Umsetzung in großtechnischem Maßstab nur bedingt möglich ist,
- die erhaltenen Ausbeuten nicht befriedigen und
- das Produkt als Isomerengemisch (E + Z) gebildet wird.

Im Hinblick auf die Verwendung von 2-(2-Methylphenyl)-3-methoxyacrylsäure-methylester (I) als Ausgangsstoff für die aus der eingangs zitierten Literatur bekannten Wirkstoffe ist ein Verfahren wünschenswert, daß großtechnisch anwendbar ist und nach dem außerdem bei guten Gesamtausbeuten weitgehend das E-Isomere erhalten wird.

Demgemäß wurde ein Verfahren zur Herstellung von 2-(2-Methylphenyl)-3-methoxyacrylsäure-methylester (I) durch Formylierung von 2-Methylphenylessigsäure-methylester (II) in einem inerten Lösungsmittel in Gegenwart einer Base, und anschließende Methylierung des gebildeten Enolats III in dem M⁺ für ein Alkalimetall-kation steht, gefunden, welches dadurch gekennzeichnet ist, daß man als inertes Lösungsmittel ein organisches aprotisch dipolares Lösungsmittel und als Base ein Alkalimetall-alkoholat verwendet.

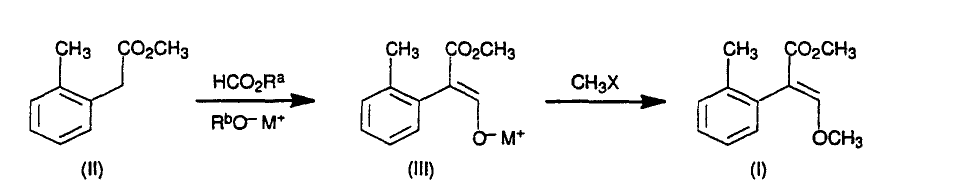

R^{a} (Ester der Ameisensäure) und R^{b} (Alkoholatrest) stehen unabhängig voneinander für Alkylgruppen, besonders C₁-C₄-Alkyl wie Methyl, Ethyl, Propyl, iso-Propyl, Butyl, sec.-Butyl, iso-Butyl und tert.-Butyl, insbesondere Methyl.

X im Methylierungsreagens steht für ein Halogenatom (insbesondere Chlor, Brom und Iod) oder ein Äquivalent einer Sulfatgruppe.

Nach dem erfindungsgemäßen Verfahren geht man im allgemeinen so vor, daß man zunächst 2-Methylphenylessigsäure-methylester (II) und den Ameisensäure-alkylester im inerten aprotisch polaren Lösungsmittel vorlegt und diese Mischung bei einer Reaktionstemperatur von -10°C bis 50°C, vorzugsweise 10°C bis 30°C, insbesondere 15°C bis 25°C, mit einer Lösung des Alkoholats im entsprechenden Alkohol versetzt.

Als inertes Lösungsmittel verwendet man nach dem erfindungsgemäβen Verfahren im allgemeinen ein organisches aprotisch polares Lösungsmittel, wobei die Art des verwendeten Lösungsmittels üblicherweise ohne Einfluß auf den Reaktionsverlauf ist. Im Hinblick auf die Aufarbeitung der Reaktionsmischung hat sich die Verwendung von Dimethylformamid, N-Methyl-pyrrolidon, Dimethylsulfoxid, N,N-Dimethylacetamid oder N-Methylformanilid, insbesondere N-Methyl-pyrrolidon, als vorteilhaft erwiesen, da sich diese Lösungsmittel leicht destillativ vom Produkt abtrennen lassen.

Die Menge des verwendeten Lösungsmittels ist ebenfalls für den Verfahrenserfolg von nur untergeordneter Bedeutung. Jedenfalls soll die Menge ausreichend sein, um eine gleichmäßige Verteilung der Reaktionspartner zu gewährleisten. Größere Mengen an Lösungsmittel stören im allgemeinen nicht sind aber aus wirtschaftlicher Sicht nicht wünschenswert. Üblicherweise verwendet man ca. 1000 ml bis 3000 ml Lösungsmittel pro mol (II), vorzugsweise 1000 ml bis 1500 ml.

Bei der erfindungsgemäßen Umsetzung wird der Ameisensäure-alkylester mindestens in molaren Mengen (1 mol pro mol II) verwendet. Da der Ameisensäure-alkylester zusätzlich als Lösungs- bzw. Verdünnungsmittel wirken kann, findet der Ameisensäure-alkylester bevorzugt in einem Überschuß von bis zu 20 mol pro mol II Verwendung. Größere Mengen an Ameisensäure-alkylester stören nicht, sind aber aus wirtschaftlicher Sicht nicht wünschenswert.

Als Alkalimetall-alkoholat finden üblicherweise Kalium- oder Natrium-Salze niedriger Alkohole, z.B. mit 1 bis 4 Kohlenstoffatomen, insbesondere Kalium- oder Natrium-methylat, Kalium- oder Natrium-ethylat, und Kalium- oder Natrium-tert.-butylat Verwendung. Aus wirtschaftlicher Sicht sind Natrium-methylat und Kalium-tert.-Butylat besonders bevorzugt. Insbesondere findet Natrium-methanolat Verwendung.

Das Alkalimetall-alkoholat wird bei der erfindungsgemäßen Umsetzung mindestens in molaren Mengen (1 mol Alkoholat pro mol II) eingesetzt. Um Einbußen bei der Ausbeute zu vermeiden hat es sich als vorteilhaft erwiesen, das Alkoholat in einem Überschuß von etwa 0,5 mol bis 3 mol, vorzugsweise 1,5 mol bis 2,5 mol, zu verwenden. Die Menge an Alkoholat sollte nicht zu hoch gewählt werden, da anderenfalls die Methylierung in der nachfolgenden Umsetzung gestört sein kann.

Das Alkoholat kann allgemein in Substanz oder als Suspension oder Lösung im entsprechenden Alkohol verwendet werden.

Nach dem erfindungsgemäßen Verfahren wird das gebildete Enolat III nicht isoliert. Die bei der Umsetzung von II mit dem Ameisensäurealkylester erhaltene Reaktionsmischung wird lediglich bei vermindertem Druck und erhöhter Temperatur von leichter flüchtigen Bestandteilen befreit. Die Temperatur hierbei sollte 130°C nicht übersteigen, da oberhalb dieser Temperatur Zersetzung (CO-Abspaltung) des Produkts beobachtet wird. Die Mindest-Temperatur richtet sich nach der Art des verwendeten Alkoholats und nach dem Druck. Im allgemeinen erfolgt die Destillation bei Drucken von 1000 bis 20 mbar, vorzugsweise 20 bis 2 mbar.

Nach den bisherigen Ergebnissen können protische Anteile (z.B. Reaktionswasser oder Alkohole) die weitere Umsetzung des Enolats zum Produkt (I) stören. Die Destillation der flüchtigeren Bestandteile sollte daher möglichst die Entfernung dieser Bestandteile sicherstellen.

Die auf etwa 2/3 bis 1/2 ihres ursprünglichen Volumens eingeengte Reaktionsmischung kann nach dem erfindungsgemäßen Verfahren ohne weitere Aufreinigung zur Methylierung eingesetzt werden. Üblicherweise geht man hierbei so vor, daß die Reaktionsmischung mit einem organischen aprotisch polaren Lösungsmittel, vorzugsweise dem bereits im ersten Umsetzungsschritt verwendeten Lösungsmittel, verdünnt wird und anschließend die verdünnte Mischung mit dem Methylierungsreagens versetzt wird.

Als organisches aprotisch polares Lösungsmittel dienen die eingangs genannten Lösungsmittel, wobei die Art des verwendeten Lösungsmittels üblicherweise ohne Einfluß auf den Reaktionsverlauf ist. Im Hinblick auf die Aufarbeitung der Reaktionsmischung hat sich ebenfalls die Verwendung von Dimethylformamid, N-Methyl-pyrrolidon, Dimethylsulfoxid, N,N-Dimethylacetamid oder N-Methylformanilid, insbesondere N-Methyl-pyrrolidon, als vorteilhaft erwiesen, da sich diese Lösungsmittel leicht destillativ vom Produkt abtrennen lassen.

Die Menge des verwendeten Lösungsmittels ist ebenfalls für den Verfahrenserfolg von nur untergeordneter Bedeutung. Jedenfalls soll die Menge ausreichend sein, um eine gleichmäßige Verteilung der Reaktionspartner zu gewährleisten. Größere Mengen an Lösungsmittel stören im allgemeinen nicht sind aber aus wirtschaftlicher Sicht nicht wünschenswert. Üblicherweise verwendet man ca. 1000 ml bis 3000 ml Lösungsmittel pro mol (II), vorzugsweise 1000 ml bis 1500 ml.

Als Methylierungsmittel eignen sich die üblichen Methylierungsreagentien, z.B. Methylhalogenide wie Methylchlorid, -bromid und iodid, sowie Dimethylformamid.

Das Methylierungsmittel wird nach dem erfindungsgemäßen Verfahren mindestens in molaren Mengen (1 mol pro mol II) verwendet. Da das Methylierungsmittel mit noch vorhandenen Resten des Alkalimetall-alkoholats reagiert, empfiehlt es sich, einen Überschuß zu verwenden. Der Überschuß richtet sich im wesentlichen nach dem Überschuß, in dem das Alkoholat in der ersten Umsetzungs-Stufe verwendet wurde. Überschüssige Mengen an Methylierungsmittel sind für die Umsetzung ohne belang und lediglich aus wirtschaftlichen Erwägungen heraus zu vermeiden.

Die Umsetzungstemperatur bei der Methylierung sollte im allgemeinen unterhalb von 50°C (bevorzugt -10 bis 50°C, besonders bevorzugt 10 bis 30°C) liegen, da oberhalb dieser Temperatur merklich Zersetzung des Enolats II oder des Acrylsäureesters (I) eintritt. Üblicherweise ist die Umsetzungstemperatur ansonsten im wesentlichen abhängig von der Art des verwendeten Methylierungsreagens. Bei der Verwendung von leichter flüchtigen Methylierungsmitteln wie Methylhalogeniden sollte die Reaktionstemperatur entsprechend niedrig gewählt sein während bei der Verwendung von schwer flüchtigen Methylierungsmitteln wie Dimethylsulfat die Reaktionstemperatur höher gewählt werden kann.

Bei der Methylierung mittels Methylchlorid hat sich eine Umsetzungstemperatur von 0°C bis 50°C, vorzugsweise 0°C bis 30°C, als vorteilhaft erwiesen.

Das erhaltene Reaktionsgemisch wird in üblicher Weise dadurch aufgearbeitet, daß man zunächst bei vermindertem Druck die flüchtigen Bestandteile weitgehend entfernt und den so erhaltenen Rückstand extraktiv reinigt. Das Produkt kann gegebenenfalls durch fraktionierte Destillation weiter gereinigt werden.

Die bei der Entfernung der flüchtigen Bestandteile zurückgewonnenen Anteile an Lösungsmittel und Reagentien können erfahrungsgemäß erneut zur Umsetzung verwendet werden.

### Verfahrensbeispiel:

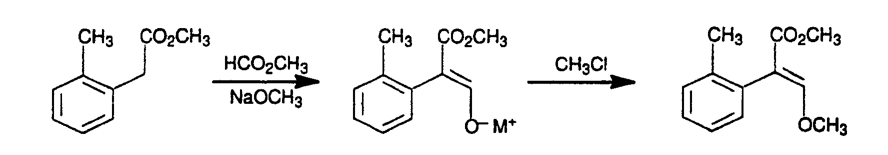

Eine Mischung von 2000 ml N-Methylpyrrolidon und 328 g (2 mol) 2-Methylphenylessigsäuremethylester wurde bei 20-25°C mit 720g Ameisensäuremethylester versetzt. Nach ca. 15 min. wurden zu der Mischung 540 g Natriummethylat-Lösung (30 %-ig in Methanol) gegeben. Nach beendeter Zugabe wurde der Druck stufenweise auf 20-2 mbar verringert und die Mischung langsam auf 80°C erwärmt, wobei eine Mischung aus Methanol, Ameisensäuremethylester und N-Methylpyrrolidon abdestillierte (insgesamt ca. 1400 ml).

Der erhaltene Rückstand wurde mit 450 ml N-Methylpyrrolidon aufgenommen und in die Mischung wurden bei 10-20°C mit 180 g (3,6 mol) Methylchlorid (gasförmig) eingeleitet. Nach ca. 12 h wurde der Methylchlorid-Überschuß und der entstandene Dimethylether bei einem Druck von 20 mbar und einer Temperatur von 50°C, und anschließend bei 2 mbar und 80°C ca 1640 g N-Methylpyrrolidon entfernt.

Der so erhaltene Rückstand wurde mit 5000 ml Wasser aufgenommen, 3 mal mit jeweils 2000 ml Toluol extrahiert. Die organische Phase wurde einmal mit 2000 ml Wasser gewaschen, getrocknet und bei vermindertem Druck (Wasserstrahlvakuum) eingeengt.

Durch fraktionierte Destillation über eine 50 cm Füllkörperkolonne erhielt man 358 g des Enolethers (Kp_{0,5}: 105-115°C, Reinheit nach GC: 99,3 %= 89 % der Theorie; E:Z-Isomere = 98:2).

## Patentansprüche

1. Verfahren zur Herstellung von 2-(2-Methylphenyl)-3-methoxyacrylsäure-methylester (I) 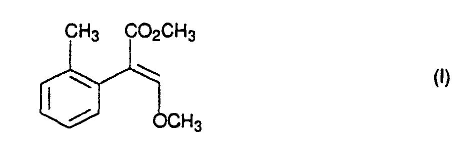 durch Formylierung von 2-Methylphenylessigsäure-methylester (II) 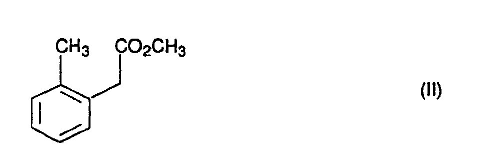 in einem inerten Lösungsmittel in Gegenwart einer Base, und anschließende Methylierung des gebildeten Enolats III 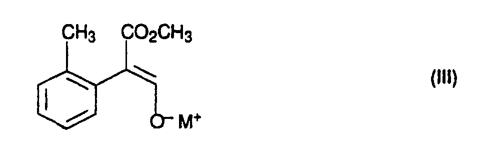 in dem M⁺ für ein Alkalimetall-kation steht, **dadurch gekennzeichnet**, daß man als inertes Lösungsmittel ein organisches aprotisch dipolares Lösungsmittel und als Base ein Alkalimetall-alkoholat verwendet.

2. Verfahren nach Anspruch 1, **dadurch gekennzeichnet**, daß man als inertes Lösungsmittel Dimethylformamid, N-Methyl-pyrrolidon, Dimethylsulfoxid, N,N-Dimethylacetamid oder N-Methylformanilid verwendet.

3. Verfahren nach Anspruch 1, **dadurch gekennzeichnet**, daß man als inertes Lösungsmittel N-Methyl-pyrrolidon verwendet.

4. Verfahren nach Anspruch 1, **dadurch gekennzeichnet**, daß man als Alkalimetall-alkoholat ein Alkalimetall-methanolat verwendet.

5. Verfahren nach Anspruch 1, **dadurch gekennzeichnet**, daß man als Alkalimetall-alkoholat ein Natrium- oder Kalium-alkoholat verwendet.

6. Verfahren nach Anspruch 1, **dadurch gekennzeichnet**, daß man als Formylierungsmittel einen Ameisensäure-C₁-C₄-alkylester verwendet.

7. Verfahren nach Anspruch 6, **dadurch gekennzeichnet**, daß man als Formylierungsmittel Ameisensäure-methylester verwendet.

8. Verfahren nach Anspruch 1, **dadurch gekennzeichnet**, daß man als Methylierungsmittel ein Methylhalogenid oder Dimethylsulfat verwendet.

9. Verfahren nach Anspruch 8, **dadurch gekennzeichnet**, daß man als Methylierungsmittel Methylchlorid, -bromid oder -iodid verwendet.

10. Verfahren nach Anspruch 1, **dadurch gekennzeichnet**, daß man die Formylierung bei Temperaturen von -10 bis 50°C durchführt.

11. Verfahren nach Anspruch 10, **dadurch gekennzeichnet**, daß man die Formylierung bei Temperaturen von 10 bis 30°C durchführt.

12. Verfahren nach Anspruch 1, **dadurch gekennzeichnet**, daß man die Methylierung bei Temperaturen von -10 bis 50°C durchführt.

13. Verfahren nach Anspruch 12, **dadurch gekennzeichnet**, daß man die Methylierung bei Temperaturen von 10 bis 30°C durchführt.

## Claims

1. A process for preparing methyl 2-(2-methylphenyl)-3-methoxy-acrylate (I) 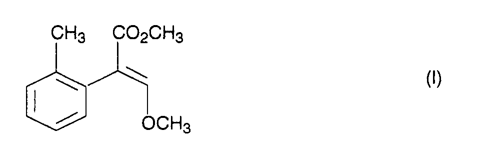 by formylation of methyl 2-methylphenylacetate (II) 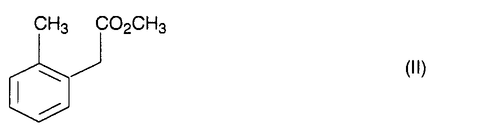 in an inert solvent in the presence of a base, and subsequent methylation of the enolate III which is formed 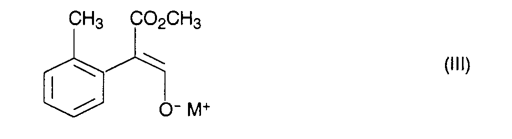 where M⁺ is an alkali metal cation, wherein an organic aprotic dipolar solvent is used as inert solvent, and an alkali metal alcoholate is used as base.

2. A process as claimed in claim 1, wherein dimethylformamide, N-methylpyrrolidone, dimethyl sulfoxide, N,N-dimethylacet-amide or N-methylformanilide is used as inert solvent.

3. A process as claimed in claim 1, wherein N-methylpyrrolidone is used as inert solvent.

4. A process as claimed in claim 1, wherein an alkali metal methanolate is used as alkali metal alcoholate.

5. A process as claimed in claim 1, wherein a sodium or potassium alcoholate is used as alkali metal alcoholate.

6. A process as claimed in claim 1, wherein a C₁_C₄_alkyl formate is used as formylating agent.

7. A process as claimed in claim 6, wherein methyl formate is used as formylating agent.

8. A process as claimed in claim 1, wherein a methyl halide or dimethyl sulfate is used as methylating agent.

9. A process as claimed in claim 8, wherein methyl chloride, bromide or iodide is used as methylating agent.

10. A process as claimed in claim 1, wherein the formylation is carried out at from -10 to 50°C.

11. A process as claimed in claim 10, wherein the formylation is carried out at from 10 to 30°C.

12. A process as claimed in claim 1, wherein the methylation is carried out at from -10 to 50°C.

13. A process as claimed in claim 12, wherein the methylation is carried out at from 10 to 30°C.

## Revendications

1. Procédé pour la préparation d'ester méthylique d'acide 2-(2-méthylphényl)-3-méthoxyacrylique (I) 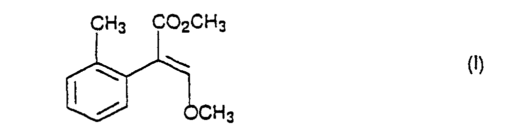 par formylation d'ester méthylique d'acide 2-méthylphénylacétique (II) 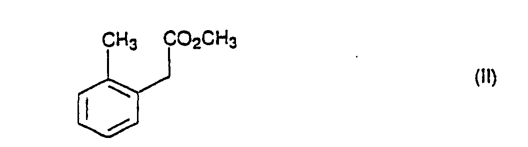 dans un solvant inerte, en présence d'une base, et méthylation subséquente de l'énolate III formé 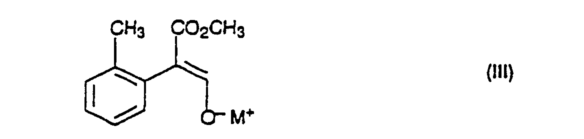 dans lequel M⁺ désigne un cation de métal alcalin, caractérisé par le fait qu'on utilise comme solvant inerte un solvant aprotique dipolaire organique et comme base un alcoolate de métal alcalin.

2. Procédé selon la revendication 1, caractérisé par le fait qu'on utilise comme solvant inerte le diméthylformamide, la N-méthyl-pyrrolidone, le diméthylsulfoxyde, le N,N-diméthylacétamide ou le N-méthylformanilide.

3. Procédé selon la revendication 1, caractérisé par le fait qu'on utilise comme solvant inerte la N-méthylpyrrolidone.

4. Procédé selon la revendication 1, caractérisé par le fait qu'on utilise comme alcoolate de métal alcalin un méthanolate de métal alcalin.

5. Procédé selon la revendication 1, caractérisé par le fait qu'on utilise comme alcoolate de métal alcalin un alcoolate de sodium ou de potassium.

6. Procédé selon la revendication 1, caractérisé par le fait qu'on utilise comme agent de formylation un ester d'alkyle en C₁-C₄ d'acide formique.

7. Procédé selon la revendication 6, caractérisé par le fait qu'on utilise comme agent de formylation l'ester méthylique d'acide formique.

8. Procédé selon la revendication 1, caractérisé par le fait qu'on utilise comme agent de méthylation un halogénure de méthyle ou sulfate de diméthyle.

9. Procédé selon la revendication 8, caractérisé par le fait qu'on utilise comme agent de méthylation le chlorure, bromure ou iodure de méthyle.

10. Procédé selon la revendication 1, caractérisé par le fait qu'on effectue la formylation à des températures de -10 à 50°C.

11. Procédé selon la revendication 10, caractérisé par le fait qu'on effectue la formylation à des températures de 10 à 30°C.

12. Procédé selon la revendication 1, caractérisé par le fait qu'on effectue la méthylation à des températures de -10 à 50°C.

13. Procédé selon la revendication 12, caractérisé par le fait qu'on effectue la méthylation à des températures de 10 à 30°C.
